# EUROPÄISCHE PATENTSCHRIFT

(11) **EP 1 335 774 B1**
(45) Veröffentlichungstag und Bekanntmachung des Hinweises auf die Patenterteilung: **09.03.2005**
(21) Anmeldenummer: 01997328.8
(22) Anmeldetag: 16.11.2001
(51) Int. Cl.: A61N 1/32, A61H 39/00, A61B 5/05

(54) **ELEKTRODENSYSTEM ZUR ELEKTRISCHEN PUNKTUAL-STIMULATIONSTHERAPIE UND HANDHABUNGSWERKZEUG HIERFÜR**
ELECTRODE SYSTEM FOR ELECTRIC POINT STIMULATION THERAPY AND A MANIPULATION TOOL THEREFOR
SYSTEME D'ELECTRODES DESTINE A LA THERAPIE DE STIMULATION PONCTUELLE ET OUTIL DE MANIPULATION DESTINE A CET EFFET

(30) Priorität: 21.11.2000 AT 8592000 U
(43) Veröffentlichungstag der Anmeldung: 20.08.2003
(73) Patentinhaber: ERNST BIEGLER GESELLSCHAFT M.B.H., 3001 Mauerbach (AT); SZELES, Josef Constantin, A-1190 Wien (AT)
(72) Erfinder: SZELES, Josef, Constantin, A-1190 Wien (AT)
(74) Vertreter: Weinzinger, Arnulf, Dipl.-Ing.
(86) Internationale Anmeldenummer: PCT/AT2001/000362
(87) Internationale Veröffentlichungsnummer: WO 2002/041943

(56) Entgegenhaltungen:
- EP-A- 0 759 307
- AT-B- 395 106
- GB-A- 2 115 700
- US-A- 3 939 841
- US-A- 5 957 862

## Beschreibung

Die Erfindung bezieht sich auf ein Elektrodensystem zur elektrischen Punktual-Stimulationstherapie mit mindestens einer bei unter der Hautoberfläche befindlichen Rezeptorbereichen anzuordnenden Punktual-Stimulationselektrode, welche eine scheibenartige Basis aufweist, die an ihrer der Hautoberfläche zuzuwendenden Seite, in der Mitte derselben, mit einer Nadel oder Spitze versehen ist, über die eine Stromeinleitung in den zu stimulierenden Rezeptorbereich erfolgt, wobei diese Punktual-Stimulationselektrode über eine flexible Leitung von einem Behandlungsstromgenerator gespeist wird. Weiter bezieht sich die Erfindung auf ein Handhabungswerkzeug zur Applikation eines derartigen Elektrodensystems an die Hautoberfläche.

Die elektrische Punktual-Stimulations-Therapie, zu der auch verschiedene Formen der Elektroakupunktur zu zählen sind, kann bei verschiedenen Gesundheitsstörungen, wie z.B. Allergien, Asthma, Adipositas, Schmerzzuständen, mit Erfolg angewendet werden. Es wird dabei häufig eine sich über mehrere Stunden oder auch über mehrere Tage erstreckende Behandlung als günstig erachtet. Dabei soll auch die Mobilität der zu behandelnden Patienten möglichst wenig eingeschränkt werden und die Behandlung nach Möglichkeit im Rahmen eines weitgehend normalen Lebensablaufes, einschließlich einer Teilnahme an Erwerbsarbeit, möglich sein. Es kommt demgemäß der Anbringung und Ausbildung von Elektroden, die bei einer derartigen Punktual-Stimulations-Therapie benützt werden, große Bedeutung zu. Im Interesse einer möglichst wirksamen Stimulation der für den jeweiligen Behandlungsfall relevanten Rezeptorenbereiche kommt einer exakten Positionierung der Elektroden an diesen Bereichen besondere Bedeutung zu. Es soll ein sicherer Sitz der Elektroden an den zu stimulierenden Rezeptorenbereichen über längere Zeiträume auch dann gewährleistet sein, wenn leichtere mechanische Kräfte unbeabsichtigt während einer Behandlung auf die Elektroden einwirken.

Bei bekannten Elektroden eingangs genannter Art (AT 395106 B) ist in der Mitte der scheibenartigen Basis an deren der Hautoberfläche zuzuwendenden Seite eine Nadel vorgesehen, welche mit einem scharfkantigen Gewinde versehen ist. Ergänzend kann auch an der der Hautoberfläche zuzuwendenden Seite der Elektrode ein Haftkleber vorgesehen sein. Das an der Nadel vorgesehene Gewinde verursacht angesichts der Kleinheit der üblicherweise vorliegenden Abmessungen der Nadel einen oft unerwünschten zusätzlichen Produktionsaufwand und verlangt auch ein feinfühliges Zielen und Drehen der Elektrode beim Einstechen an einem zu stimulierenden Rezeptorenbereich, wobei sich beim Vorhandensein eines Haftklebers an der scheibenartigen Basis auch gegen Ende des Einsetzvorganges bzw. Einstechvorganges einer solchen Elektrode eine unerwünschte Konkurrenz zwischen der beim Einstechen der Nadel der Elektrode vorzunehmenden Drehbewegung und einem bereits beginnenden Haften des Haftklebers an der Hautoberfläche ergeben kann.

Es ist ein Ziel der vorliegenden Erfindung ein Elektrodensystem eingangs erwähnter Art zu schaffen, welches eine sehr einfache Manipulation beim Ansetzen an einen zu stimulierenden Rezeptorbereich ermöglicht, wobei auch die Lokalisation eines geeigneten Rezeptorenbereiches und ein möglichst exaktes Positionieren der Stimulationselektrode am lokalisierten Rezeptorbereich auf einfache Weise möglich sein soll, und weiter auch ein stabiler Halt von positionierten Elektroden sichergestellt sein soll.

Das erfindungsgemäße Elektrodensystem eingangs erwähnter Art ist dadurch gekennzeichnet, dass zur Fixierung der jeweiligen Punktual-Stimulationselektrode an der Hautoberfläche ein vor dem Ansetzen bzw. Einstechen der Elektrode an der Hautoberfläche anzuordnendes Ringplättchen vorgesehen ist, das mindestens an der der Hautoberfläche zuzuwendenden Seite eine zur Haftung an der Hautoberfläche vorgesehene Klebeschicht trägt, und dass weiter eine Haftverbindung zwischen der anderen Seite dieses Ringplättchens und der die Nadel oder Spitze aufweisenden Seite der Basis der Punktual-Stimulationselektrode vorgesehen ist. Durch diese Ausbildung des Elektrodensystems kann der vorstehend angeführten Zielsetzung gut entsprochen werden. Es kann dadurch, dass das Ringplättchen separat vor dem Ansetzen bzw. Einstechen der Elektrode an der Hautoberfläche angeordnet wird, dieses Ringplättchen ohne Rücksichtnahme auf allfällige Erfordernisse, die sich bei einem Einstechen der Elektrode ergeben, platziert und satt an die Hautoberfläche angedrückt werden, so dass sich auf einfache Weise eine gute Klebehaftung erzielen lässt. Weiter ist auch durch die im Ringplättchen vorhandene Öffnung die Möglichkeit einer sehr exakten Positionierung dieses Ringplättchens am zu stimulierenden Rezeptorbereich geschaffen, wobei es insbesondere möglich ist, einen Suchstift oder eine Suchelektrode durch die im Ringplättchen vorhandene Öffnung hindurchzustecken und im Überstreichen der Hautoberfläche mit dem Suchstift bzw. der Suchelektrode die Lage von Rezeptorenbereichen festzustellen, wobei beim Erreichen eines Rezeptorbereiches das Ringplättchen passgenau an die Hautoberfläche angedrückt werden kann. Der zur Stimulation vorgesehene Rezeptorbereich ist durch das an die Hautoberfläche geklebte Ringplättchen klar gekennzeichnet, und es kann damit die anzuordnende Punktual-Stimulationselektrode bei ihrem Anordnen bzw. Einstechen an diesen Rezeptorbereich sehr einfach und genau positioniert werden, und es ist darüber hinaus durch das Ringplättchen eine klar definierte Auflage geschaffen, an der die scheibenartige Basis der Punktual-Stimulationselektrode beim Anbringen bzw. Einstechen dieser Elektrode zum Anliegen kommt, und mit einer Haftverbindung eine sichere Fixierung erhält, ohne dass sich dabei Unregelmäßigkeiten der Hautoberfläche störend auswirken. Als Haftverbindung wird dabei vorzugsweise eine Klebeverbindung vorgesehen, welche z.B. dadurch realisiert werden kann, dass man auch an der von der Hautoberfläche abgewandten Fläche des Ringplättchens eine Klebeschicht vorsieht. Im Prinzip kommen aber auch andere Haftverbindungen in Betracht, wie z.B. ein Aneinanderhalten von Ringplättchen und Elektrodenbasis durch magnetische oder elektrostatische Kräfte. Beim Einsatz einer Klebeschicht zur Bildung der Haftverbindung kann man verschiedene Maßnahmen vorsehen, um einerseits die Haftwirkung an der von der Hautoberfläche abgewandten Seite des Ringplättchens beim Andrücken des Ringplättchens an die Hautoberfläche möglichst nicht störend in Erscheinung treten zu lassen, und dennoch eine gute Fixierung der Basis der Elektrode am Ringplättchen zu erzielen. So kann man am Ringplättchen eine Klebeschicht vorsehen und das Andrücken des Ringplättchens mit einem weitgehend klebeinerten Werkzeug vornehmen oder eine solche am Ringplättchen vorgesehene Klebeschicht zunächst mit einer nach dem Andrücken des Ringplättchens abzuziehenden Schutzfolie überdecken; andere Varianten sehen das Anbringen einer entsprechenden Klebeschicht an der dem Ringplättchen zuzuwendenden Seite der Basis der Elektrode oder das Vorsehen spezieller Schichten, welche im Wesentlichen nur aneinander haften, nicht jedoch an anderen Körpern, am Ringplättchen und an der Basis der Elektrode vor.

Für ein einfaches und exaktes Handhaben der beim erfindungsgemäß ausgebildeten Elektrodensystem vorgesehenen Stimulationselektrode beim Ansetzen bzw. Einstechen derselben ist es vorteilhaft, wenn man vorsieht, dass die scheibenartige Basis der Punktual-Stimulationselektrode an ihrer der Nadel oder Spitze abgewandten Seite eine zum passenden Einfügen in eine Aufnahmeöffnung eines Handhabungswerkzeuges und zum Aufsetzen einer elektrischen Verbindungsklemme vorgesehene Erhebung aufweist. Man kann solcherart mit einem verhältnismäßig einfach ausgebildeten Werkzeug eine gute Seitenführung der Elektrode beim Ansetzen bzw. Einstechen und damit auch eine gute Positionierung an der bereits durch das Ringplättchen definierten Stelle erzielen. Weiter ist durch diese Erhebung ein den Haftsitz der Elektrode am Ringplättchen nicht beeinflussender Ansatzpunkt für eine elektrische Verbindungsklemme, die eine flexible Leitung an die Elektrode anschließt, geschaffen, wobei eine solche Verbindungsklemme z.B. in Art eines Druckknopfes oder in Art einer C-Feder ausgebildet sein kann. Es ist dabei weiter für einen möglichst exakten Sitz der Punktual-Stimulationselektrode in einem mit einer Aufnahmeöffnung versehenen Werkzeug günstig, wenn man vorsieht, dass die an der Basis der Punktual-Stimulationselektrode vorgesehene Erhebung einen ringsum laufenden ausgeprägten Seitenrand aufweist, der durch zueinander parallele erzeugende Gerade, die annähernd senkrecht zur Basis verlaufen, gebildet ist oder zumindest in Teilbereichen hinterschnitten geformt ist. Die erwähnte hinterschnittene Ausbildung ist dabei insbesondere für das Aufsetzen einer elektrischen Verbindungsklemme von Vorteil.

Um einen noch weiter gehenden Schutz des Elektrodensystems gegenüber unerwünschten, von außen kommenden Einflüssen auch über längere Zeiträume zu erzielen, ist es vorteilhaft, wenn man vorsieht, dass eine Überdeckung der jeweiligen Punktual-Stimulationselektrode, welche mit einem klebenden Ringplättchen an der Hautoberfläche fixiert ist, mit einer flexiblen, rings um diese Elektrode an der Hautoberfläche haftenden Pflasterscheibe vorgesehen ist. Hierbei ist es weiter günstig, und zwar sowohl für den Halt der Pflasterscheibe an der Hautoberfläche als auch für die Führung der an die jeweilige Elektrode angeschlossenen Leitung, wenn die Pflasterscheibe einen annähernd radialen Einschnitt zur Passage der zum Behandlungsstromgenerator führenden Leitung aufweist.

Das erfindungsgemäß ausgebildete Handhabungswerkzeug, welches zur Applikation eines wie vorerwähnt ausgebildeten Elektrodensystems vorgesehen ist, ist gekennzeichnet durch einen in einem Hohlstabgehäuse, das einem Handschreibgerät formähnlich und an seinem Vorderende zugespitzt geformt ist sowie an diesem Vorderende eine Öffnung aufweist, axial verschiebbar gelagerten, elektrisch leitfähigen Stift, der über einen Teil seiner Länge ausschiebbar und durch die Öffnung rückziehbar ist, und dessen Durchmesser an der am Vorderende des Gehäuses vorgesehenen Öffnung dem Innendurchmesser des Ringplättchens entspricht, wobei zum Ausschieben und Rückziehen des elektrisch leitfähigen Stiftes im Hohlstabgehäuse ein mit mindestens einem am Hohlstabgehäuse vorgesehenen Betätigungselement steuerbarer Axialverschiebemechanismus, mit dem dieser Stift gekuppelt ist, angeordnet ist, wobei dieser Stift im Werkzeug ohne direkte elektrisch leitende Verbindung zu an der Gehäuse-oberfläche vorgesehenen elektrisch leitfähigen Körpern angeordnet und über eine im Werkzeug angeordnete Schaltungsanordnung zur Messung des elektrischen Widerstandswertes mit einem solchen elektrisch leitfähigen Körper verbunden ist, welche Schaltungsanordnung den Wert des elektrischen Widerstandes des jeweils außen zwischen dem elektrisch leitfähigen Stift und dem genannten elektrisch leitfähigen Körper anliegenden Stromweges erfasst, und wobei diese Schaltungsanordnung mit einem optischen und/oder akustischen Indikator verbunden ist. Es kann mit diesem Werkzeug das Elektrodensystem auf sehr einfache Weise an den zu stimulierenden Rezeptorbereichen platziert und fixiert werden, wobei durch elektrische Widerstandsmessung im Zuge des Überstreichens in Betracht kommender Bezirke der Hautoberfläche entsprechende Stellen ermittelt werden und an diesen Stellen sogleich ein zuvor mit dem Handhabungswerkzeug aufgenommenes Ringplättchen angedrückt bzw. fixiert werden kann, wonach auf die solcherart markierte und vorbereitete Stelle die Stimulationselektrode angebracht bzw. dort eingestochen werden kann, was gleichfalls mit diesem Handhabungswerkzeug vorgenommen werden kann. Das Erreichen eines Rezeptorbereiches im Zuge des Überstreichens eines in Frage kommenden Bezirkes der Hautoberfläche wird dabei mit einem optischen und/oder akustischen Indikator signalisiert, welcher vorteilhafterweise im Gehäuse des Handhabungswerkzeuges in entsprechend erkennbarer Weise angeordnet ist. Es ist dabei für das Aufnehmen des Ringplättchens mit dem Handhabungswerkzeug und für das Anbringen des Ringplättchens an der Hautoberfläche vorteilhaft, dass der Durchmesser des elektrisch leitfähigen, axial verschiebbar gelagerten Stiftes an der am Vorderende des Gehäuses des Handhabungswerkzeuges vorgesehenen Öffnung dem Innendurchmesser des Ringplättchens entspricht. Es wird auf diese Weise auch erreicht, dass das Ringplättchen während des Suchens der Lage von Rezeptorbereichen durch Überstreichen der Hautoberfläche mit dem elektrisch leitfähigen Stift auf diesem Stift ohne weitere zusätzliche Maßnahmen aufgesteckt verharrt und jederzeit ohne Weiteres an die Hautoberfläche angedrückt werden kann. Hierbei kann man das Ringplättchen mit dem Rand der Öffnung, durch die der genannte leitfähige Stift über einen Teil seiner Länge ausschiebbar ist, an die Hautoberfläche andrücken. Hierfür wird der Stift in die Öffnung eingeschoben bzw. zurückgezogen, wobei dieser Vorgang mit dem steuerbaren Axialverschiebemechanismus, mit dem dieser Stift gekuppelt ist, bewirkt werden kann oder auch selbsttätig dadurch stattfinden kann, dass der elektrisch leitfähige Stift nicht nur mit dem steuerbaren Axialverschiebemechanismus gekuppelt ist, sondern auch in Axialrichtung federnd verschiebbar angeordnet ist, so dass dieser Stift durch entsprechendes Andrücken des Handhabungswerkzeuges an die Hautoberfläche gegen die Federkraft in die ihn umgebende Öffnung rückschiebbar ist, wobei das Ringplättchen in Axialrichtung vom Stift herunter gleitet und vom Rand der den Stift umgebenden Öffnung an die Hautoberfläche angepresst wird.

Vorteilhafterweise wird die am Handhabungswerkzeug vorgesehene Öffnung, aus der der elektrisch leitfähige Stift ausschiebbar ist, auch zur Aufnahme und Halterung von Elektroden ausgebildet, welche, wie oben erläutert, an der der Nadel abgewandten Seite der scheibenartigen Basis mit einer Erhebung versehen sind. Eine diesbezügliche Ausführungsform des erfindungsgemäßen Handhabungswerkzeuges ist dadurch gekennzeichnet, dass der Querschnitt der am Vorderende des Gehäuses des Handhabungswerkzeuges vorgesehenen Öffnung zum Einfügen der an der scheibenartigen Basis der Punktual-Stimulationselektrode vorgesehenen Erhebung bemessen ist. Es ist dabei zum Aufnehmen einer Elektrode der elektrisch leitfähige Stift zunächst mit dem Axialverschiebemechanismus so weit zurückzuziehen, dass die an der Elektrode vorgesehene Erhebung in die Öffnung eingeführt werden kann. Das Ansetzen bzw. Einstechen der Elektrode an bzw. in die Hautoberfläche kann danach durch Druckausübung von Hand aus, wobei die Elektrode vom Rand der Öffnung gedrückt wird, oder unter Zuhilfenahme des Axialverschiebemechanismus, der den elektrisch leitfähigen Stift an die zunächst in der Öffnung sitzende Erhebung der Elektrode andrückt, vorgenommen werden.

Bei einer federnd verschiebbaren Anordnung des elektrisch leitfähigen Stiftes, der wir vorstehend erwähnt, nicht nur als Suchelektrode zur Positionsbestimmung von Rezeptorbereichen dient, sondern auch verschiedene Funktionen beim Anbringen des Ringplättchens und der Elektrode haben kann, ist es von diesen Funktionen her gesehen und auch aus baulichen Gründen vorteilhaft, wenn man vorsieht, dass zur axialen Federung und zur Axialverschiebung des axial verschiebbar gelagerten Stiftes eine erste Feder, welche mit ihrem einen Ende an einem ersten Fixpunkt im Werkzeug sitzt und mit ihrem anderen Ende an diesem Stift angreift, und weiter eine aus einer zweiten Feder und einem Axialverschiebemechanismus gebildete Reihenanordnung vorgesehen sind, wobei ein Ende dieser Reihenanordnung an einem zweiten Fixpunkt im Werkzeug sitzt und das andere Ende dieser Reihenanordnung am genannten Stift angreift. Es ergibt sich dabei weiter eine baulich günstige Lösung, wenn man vorsieht, dass eine erste Feder, welche den axial verschiebbar gelagerten Stift an seinem Vorderabschnitt umgibt und mit ihrem einen Ende an einem Fixpunkt in der Nähe der Öffnung sitzt und mit ihrem anderen Ende am genannten Stift angreift, vorgesehen ist, dass auf den hinteren Abschnitt des Stiftes eine aus dem Axialverschiebemechanismus, einer Batterie und einer zweiten Feder gebildete Reihenanordnung folgt, wobei die zweite Feder an einem zweiten Fixpunkt im Werkzeug sitzt und auch eine elektrische Verbindung zu einem Pol der Batterie bildet.

Hinsichtlich des Axialverschiebemechanismus kann eine baulich einfache, rein mechanische Lösung dadurch erhalten werden, dass man vorsieht, dass der Axialverschiebemechanismus zwei Stützkörper aufweist, die in Axialrichtung des federnd axial verschiebbar gelagerten Stiftes auf diesen Stift folgend in axialer Aufeinanderfolge aneinander anliegend und gegeneinander verdrehbar angeordnet sind und schräg zur Axialrichtung verlaufende Flächen aufweisen, wobei Schrägflächen des einen Stützkörpers an Schrägflächen des anderen Stützkörpers anliegen und wobei die axiale Gesamtlänge dieser beiden Stützkörper durch relatives Verdrehen veränderbar ist und einer dieser Stützkörper im Werkzeug drehfest angeordnet ist, während der andere Stützkörper verdrehbar angeordnet und mit einem außen am Gehäuse angeordneten Betätigungselement zum Verdrehen dieses Stützkörpers verbunden ist. Hinsichtlich der Axialverschiebung des elektrisch leitfähigen Stiftes, welche Verschiebung, wie oben erörtert für die Funktion dieses Stiftes als Suchelektrode und auch für das Anbringen des Ringplättchens und der Elektrode bedeutsam ist, kann eine baulich einfache, sanft arbeitende Ausführungsform des erfindungsgemäßen Handhabungswerkzeuges erhalten werden, wenn man vorsieht, dass am axial verschiebbar gelagerten Stift zwei axial wirkende Federn angreifen, welche sich mit ihren der jeweiligen Angriffsstelle am Stift abgewandten Enden je an einem gegen Axialverschiebung festliegenden Fixpunkt im Gehäuse abstützen, dass der Axialverschiebemechanismus durch einen am Stift seitlich abstehenden Ansatz und einen quer zur Axialrichtung des Stiftes bewegbaren Schiebekörper gebildet ist, wobei am Ansatz und/oder am Schiebekörper mindestens eine Schrägfläche vorgesehen ist, durch welche der Stift bei einer quer zur Axialrichtung des Stiftes erfolgenden Bewegung des Schiebekörpers im Zusammenwirken von Ansatz und Schiebekörper aus einer Ruhelage in Richtung eines Ausschiebens aus der Öffnung bewegbar ist, und dass das Vorderende des Gehäuses des Werkzeuges durch eine gegenüber dem übrigen Gehäuseabschnitt axial versetzbar angeordnete Kappe gebildet ist, wobei durch diese axiale Versetzung die axiale Relativlage der Öffnung und des Vorderendes des Stiftes dahingehend veränderbar ist, dass in einer Position der Kappe der in Ruhelage befindliche Stift ein Stück aus der Öffnung ragt und in einer anderen Position der Kappe der in Ruhelage befindliche Stift ein Stück gegenüber dem Außenrand der Öffnung zurückgezogen ist. Hierbei ist es hinsichtlich einer baulich einfachen und leicht betätigbaren Lösung für die Axialversetzung der Kappe vorteilhaft, wenn man vorsieht, dass die axial versetzbar angeordnete Kappe zur Axialversetzung gegenüber dem übrigen Gehäuseabschnitt schraubbar angeordnet ist. Zur einfachen Betätigung des Werkzeuges ist es dabei weiter günstig, wenn der Schiebekörper mit einem das Betätigungselement bildenden Druckknopf versehen ist.

Eine besonders sanft arbeitende Funktion des Axialverschiebemechanismus kann dadurch erhalten werden, dass als Axialverschiebemechanismus ein elektromotorisch oder elektromagnetisch angetriebenes Verschiebegetriebe vorgesehen ist, welches über elektrische Leichtgangtasten oder Sensorfelder steuerbar ist. Bei einer solchen Ausführungsform kann auch der Einfluss von Kräften, die zur Steuerung des Axialverschiebemechanismus auf ein Betätigungselement desselben auszuüben sind, weitestgehend ausgeschaltet werden, und es kann gewünschtenfalls, auch ohne federnde Anordnung des elektrisch leitfähigen Stiftes, eine feinfühlig, den jeweiligen Erfordernissen entsprechende Bewegung dieses Stiftes erzielt werden, was insbesondere beim Einstechen der Elektrode in die Hautoberfläche vorteilhaft ist.

Die Erfindung wird nun anhand von Beispielen und unter Bezugnahme auf die schematisch gehaltene Zeichnung weiter erläutert. In der Zeichnung zeigt
Fig. 1 ein erfindungsgemäß ausgebildetes Elektrodensystem im Schnitt, und es zeigen die
Fig. 2, 3 und 4 verschiedene Stadien bei der Herstellung eines derartigen Elektrodensystems in schematisierter Schnittdarstellung.
Die Fig. 5 und 6 zeigen, gleichfalls im Schnitt, Varianten beim Einstechen der Stimulationselektrode eines derartigen Elektrodensystems, und
die Fig. 7 und 8 zeigen das Vorsehen elektrischer Anschlüsse bei derartigen Elektroden.
Fig. 9 zeigt eine weitere Ausführungsform eines erfindungsgemäß ausgebildeten Elektrodensystems im schematisierten Schnitt und
Fig. 10 dieses Elektrodensystem in Draufsicht, und es zeigen
die Fig. 11 und 12 eine Variante zum Elektrodensystem der Fig. 9 und 10.
Fig. 13 zeigt eine Ausführungsform eines erfindungsgemäßen Handhabungswerkzeuges zur Positionierung, Bildung und Applikation eines erfindungsgemäß ausgebildeten Elektrodensystems in einer Ansicht, und
Fig. 14 zeigt dieses Handhabungswerkzeug in einer Explosionsdarstellung.
Die Fig. 15 und 16 zeigen eine andere Ausführungsform eines derartigen, erfindungsgemäß ausgebildeten Handhabungswerkzeuges in einer Ansicht und einer teilweisen Explosionsdarstellung.

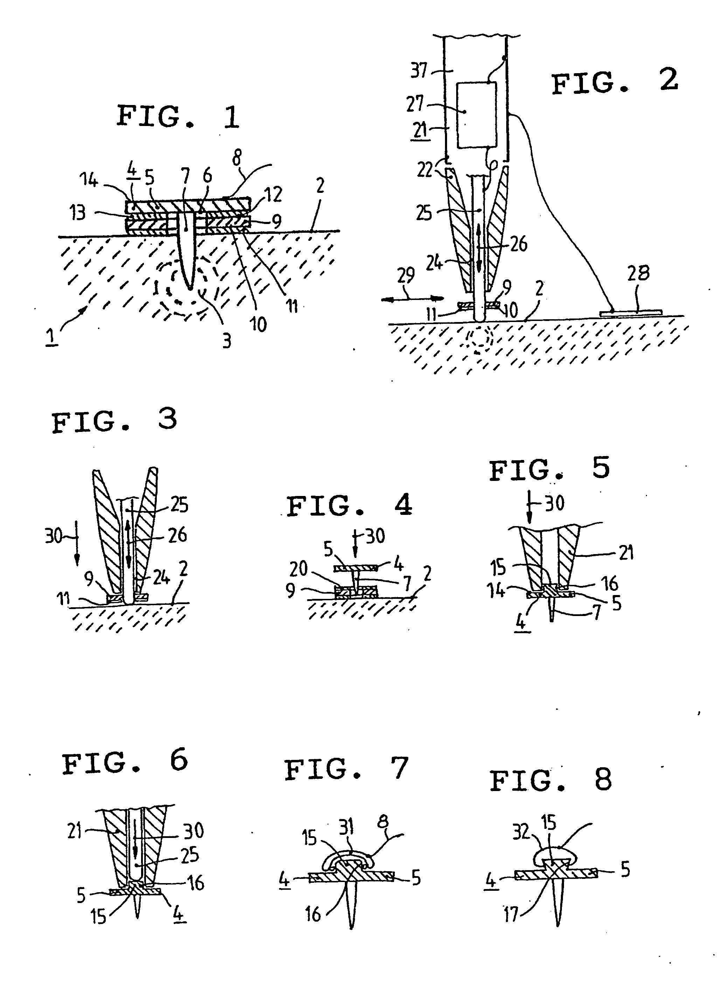

Die in Fig. 1 grob schematisch im Schnitt dargestellte Ausführungsform eines erfindungsgemäß ausgebildeten Elektrodensystems 1, welches zur elektrischen Punktual-Stimulations-Therapie vorgesehen ist, hat eine Punktual-Stimulationselektrode 4, welche bei unter der Hautoberfläche 2 befindlichen Rezeptorbereichen 3 anzuordnen ist. Die Stimulationselektrode 4 weist eine scheibenförmige Basis 5 auf, die an ihrer der Hautoberfläche zuzuwendenden Seite 6, in der Mitte derselben, mit einer in die Hautoberfläche 2 einzustechenden Nadel 7 versehen ist. Über die Stimulationselektrode 4 erfolgt eine Stromeinleitung in den zu stimulierenden Rezeptorbereich 3, und es wird hierzu die Stimulationselektrode 4 über eine flexible Leitung 8 von einem nicht näher dargestellten Behandlungsstromgenerator gespeist. Zur Fixierung der Punktual-Stimulationselektrode 4 an der Hautoberfläche 2 ist ein vor dem Einstechen der Elektrode 4 an der Hautoberfläche 2 anzuordnendes Ringplättchen 9 vorgesehen, welches an der der Hautoberfläche zuzuwendenden Seite 10 eine zur Haftung an der Hautoberfläche vorgesehene Klebeschicht 11 trägt, und es ist weiter eine Haftverbindung 13 zwischen der anderen Seite 12 des Ringplättchens 9 und der die Nadel 7 aufweisenden Seite 6 der scheibenartigen Basis 5 der Stimulationselektrode 4 vorgesehen.

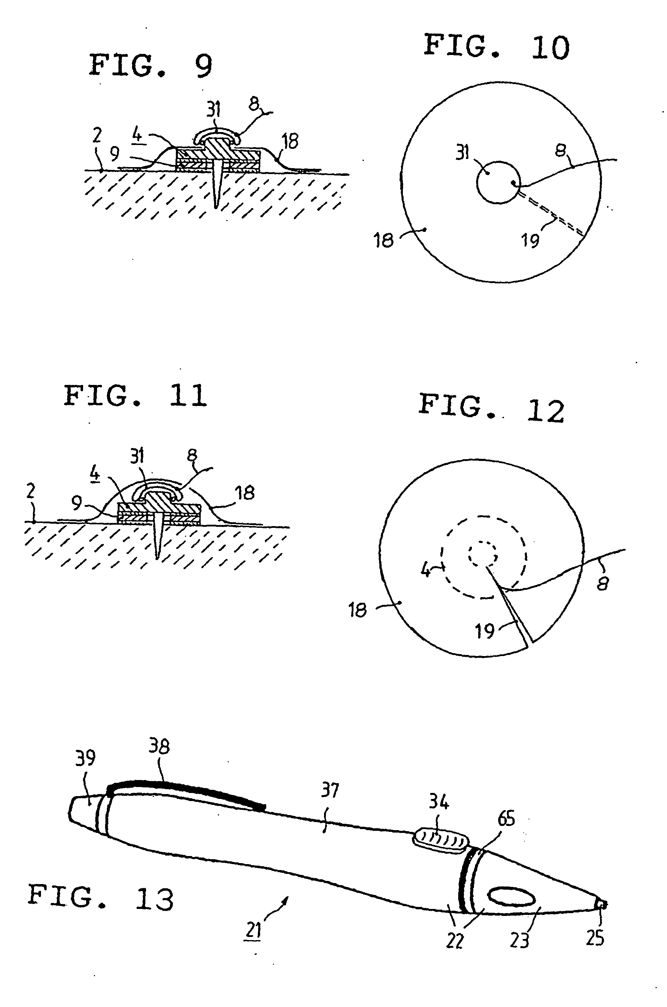

Zur Bildung bzw. Applikation eines wie in Fig. 1 dargestellt ausgebildeten Elektrodensystems wird vorzugsweise so vorgegangen, dass man zunächst für das Vorhandensein von Rezeptorbereichen in Frage kommende Zonen der Hautoberfläche hinsichtlich des Vorliegens örtlicher Abweichungen des elektrischen Widerstandswertes untersucht und dann an solcherart gefundenen Stellen, bei denen sich Rezeptorenbereiche unter der Hautoberfläche befinden, den Aufbau bzw. die Applikation eines erfindungsgemäß ausgebildeten Elektrodensystems vornimmt. Man kann hierzu, wie in Fig. 2 schematisch dargestellt ist, ein Handhabungswerkzeug 21 verwenden, welches einen elektrisch leitfähigen Stift 25 aufweist, der in einem Gehäuse 22 des Werkzeuges 21 in Axialrichtung 26 verschiebbar und ohne direkte elektrisch leitende Verbindung zu elektrisch ausgebildeten Teilen des Gerätegehäuses 22 angeordnet ist. Im Werkzeug 21 ist auch eine Schaltungsanordnung 27 angeordnet, welche zur Messung des elektrischen Widerstandswertes eines Körpers dient, der zwischen dem elektrisch leitfähigen Stift 25 und einem elektrisch leitfähig ausgebildeten Teil 37 des Gehäuses 22 außen anliegt. Hierzu ist die Schaltungsanordnung 27 einerseits an den Stift 25 und andererseits an den Gehäuseteil 37 angeschlossen. Es wird nun von einem das Werkzeug 21 handhabenden Therapeuten, der dabei den Gehäuseteil 37 berührt, durch bloßen Handkontakt oder durch Verwendung einer Hilfselektrode 28 eine elektrische Verbindung zur Hautoberfläche 2 eines zu untersuchenden Patienten hergestellt und der Stift 25, wie durch den Doppelpfeil 29 angedeutet ist, hin- und hergehend über die Hautoberfläche 2 des Patienten geführt, bis eine Stelle der Hautoberfläche gefunden ist, an der eine für das Vorliegen eines Rezeptorbereiches charakteristische Änderung des Wertes des elektrischen Widerstandes erkennbar ist, wobei eine solche Veränderung des elektrischen Widerstandswertes mit einem nicht näher dargestellten Indikator der Schaltungsanordnung 27 angezeigt wird. Ist eine solche Stelle der Hautoberfläche 2, unter der sich ein Rezeptorbereich 3 befindet, gefunden, wird ein Ringplättchen 9, welches zuvor auf den Stift 25 aufgesteckt worden ist und an seiner der Hautoberfläche 2 zugewandten Seite 10 eine Klebeschicht 11 trägt, an die Hautoberfläche 2 angedrückt, wobei dieses Andrücken, wie Fig. 3 zeigt, mit dem Rand der Öffnung 24 des Werkzeuges 21 vorgenommen werden kann. Im Zuge dieses Andrückvorganges wird der zuvor aus der Öffnung 24 ausgeschoben gewesene Abschnitt des Stiftes 25 in die Öffnung eingeschoben, wodurch der Stift 25 aus der Öffnung des Ringplättchens 9 gleitet und das Ringplättchen an der Hautoberfläche 2 durch Andruck des Werkzeuges 21 in Richtung des Pfeiles 30 fixiert wird. Das Rückschieben des Stiftes 25 erfolgt bei einer in Axialrichtung 26 federnden Lagerung des Stiftes 25 selbsttätig oder kann mit einem geeigneten Axialverschiebemechanismus, an den der Stift 25 angekoppelt ist, herbeigeführt werden. Sobald das Ringplättchen 9 an der Hautoberfläche 2 fixiert ist, wird eine Punktual-Stimulationselektrode, welche eine scheibenartige Basis 5 aufweist, wie Fig. 4 zeigt, zum Einstich der Nadel 7 dieser Elektrode in eine am Rezeptorbereich 3 gelegene Hautstelle entsprechend dem dort bereits fixierten Ringplättchen 9 positioniert und an das Ringplättchen angedrückt, wie durch den Pfeil 30 angedeutet ist. Es ist dabei in dem in Fig. 4 dargestellten Fall ein Ringplättchen 9 verwendet, welches an der der Elektrode 4 zugewandten Seite mit einer Klebeschicht 20 versehen ist, welche dann, sobald die Elektrode 4 an das Ringplättchen 9 angedrückt ist, eine Haftverbindung (Fig. 1) zwischen der der Elektrode 4 zugewandten Seite 12 des Ringplättchens 9 und der die Nadel 7 aufweisenden Seite 6 der Basis 5 der Elektrode 4 bildet. Wie bereits oben erwähnt, sind auch andere Varianten zur Herstellung einer solchen Haftverbindung 13 gangbar, wie z.B. dass man an der dem Ringplättchen 9 zuzuwendenden Seite 6 der Basis 5 der Elektrode 4 eine Klebeschicht vorsieht. Um einen guten, gegen unbeabsichtigtes seitliches Verschieben der Stimulationselektrode gesicherten Sitz derselben in einem Handhabungswerkzeug zu erzielen und um auch eine leicht handhabbare Befestigungsstelle für den elektrischen Anschluss einer Leitung an die jeweilige Elektrode zu schaffen, sieht man vorteilhaft an der Basis 5 der Elektrode 4 an ihrer der Nadel 7 abgewandten Seite 14 eine Erhebung 15 vor, welche zum passenden Einfügen in eine Aufnahmeöffnung eines Handhabungswerkzeuges geformt ist, wie dies aus den Fig. 5 bis 8 ersehen werden kann. Die Abmessungen werden dabei vorzugsweise so gewählt, dass sich ein leicht haltender Schiebesitz einer derartigen Erhebung in der Aufnahmeöffnung des Handhabungswerkzeuges ergibt. Das Applizieren der Stimulationselektrode 4 kann dabei, wie in Fig. 5 dargestellt ist, durch entsprechendes Andrücken der Elektrode mit einem Werkzeug 21 an das zuvor an der Hautoberfläche fixierte Ringplättchen erfolgen oder, wie in Fig. 6 dargestellt ist, durch Ausschieben der mit einer im Werkzeug 21 sitzenden Erhebung 15 versehenen Stimulationselektrode 4 mit einem in geeigneter Weise im Werkzeug verschiebbaren Stift 25, der den entsprechend dem Pfeil 30 wirkenden Druck auf die Elektrode zum Ausschieben derselben aus dem Werkzeug 21 und zum Anpressen an das Ringplättchen liefert. Für das Erzielen des erwähnten Schiebesitzes ist es günstig, wenn die Erhebung 15 einen ringsum laufenden ausgeprägten Seitenrand 16 aufweist, der durch zueinander parallele erzeugende Geraden, die annähernd senkrecht zur Basis 5 verlaufen, gebildet ist. Bei einer leichten Abrundung ergibt sich dabei auch ein guter Sitz für einen druckknopfartigen Anschluss 31 einer zur Elektrode führenden Leitung 8 (Fig. 7). Sieht man zum Anschluss einer derartigen Leitung 8 eine einfache Federklemme 32 vor, kann es vorteilhaft sein, den Seitenrand 16 der Erhebung 15 in Teilbereichen mit Hinterschneidungen 17 zu formen, wie dies in Fig. 8 dargestellt ist.

Bei der in Fig. 9 und 10 dargestellten Ausführungsform eines erfindungsgemäß ausgebildeten Elektrodensystems ist eine Überdeckung der Punktual-Stimulationselektrode 4, welche mit einem klebenden Ringplättchen 9 an der Hautoberfläche 2 fixiert ist, mit einer Pflasterscheibe 18 vorgesehen, welche flexibel ausgebildet ist und rings um die Elektrode 4 an der Hautoberfläche 2 haftet. Diese Pflasterscheibe 18 weist eine mittige Öffnung auf, durch welche die Erhebung 15 nach außen ragt. Auf diese Erhebung 15 ist ein Anschluss 31, eine Federklemme oder eine ähnliche Verbindung für eine zur Elektrode 4 führende Leitung 8 aufgesetzt. Zur Erleichterung der Anbringung des Pflasters kann man eventuell, wie in Fig. 10 strichliert dargestellt ist, einen annähernd radialen Einschnitt 19 im Pflaster 18 vorsehen. Die Pflasterscheibe 18 schützt auf sehr wirksame Weise das Elektrodensystem gegen störende Einflüsse von außen. Bei der in den Fig. 11 und 12 dargestellten Variante überdeckt die Pflasterscheibe 18 die Elektrode 4 und den an dieser Elektrode angebrachten Anschluss 31, und es ist die von der Elektrode 4 zu einem nicht näher dargestellten Behandlungsstromgenerator führende Leitung 8 durch einen in der Pflasterscheibe 18 vorgesehenen, annähernd radialen Einschnitt 19 gezogen, wie aus Fig. 12 ersehen werden kann.

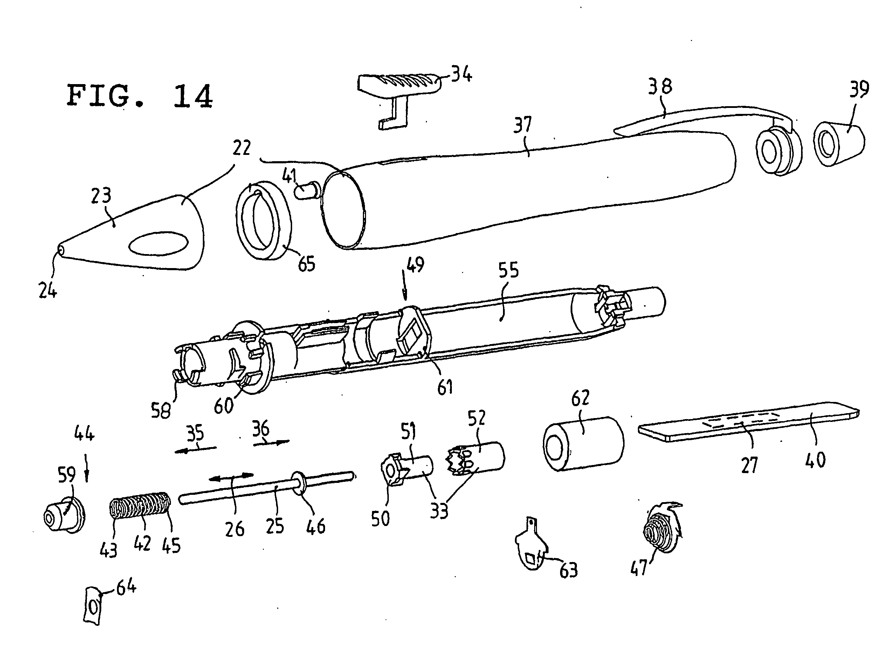

Das in den Fig. 13 und 14 dargestellte Ausführungsbeispiel eines erfindungsgemäßen Handhabungswerkzeuges 21 weist ein einem Handschreibgerät formähnliches Hohlstabgehäuse 22 auf, welches an seinem vorderen Ende durch eine aus Isoliermaterial bestehende Kappe 23 und im übrigen Teil durch ein Gehäuserohr 37 gebildet ist. Die das Vorderende bildende Kappe 23 weist eine Öffnung 24 auf, durch die ein im Gehäuse 22 axial verschiebbar gelagerter elektrisch leitfähiger Stift 25 über einen Teil seiner Länge im Sinne des Pfeiles 35 ausschiebbar und auch durch diese Öffnung 24 im Sinne des Pfeiles 36 rückziehbar ist. Der Stift 25 ist mit einem Axialverschiebemechanismus 33 gekuppelt, der mit einem Betätigungselement 34, welches am Hohlstabgehäuse 22 außen angeordnet ist, gesteuert werden kann, um den Stift 25 in seiner durch den Doppelpfeil 26 dargestellten Axialrichtung zu verschieben. Es kann dabei der Stift 25, wie aus Fig. 11 ersichtlich, mit dem Axialverschiebemechanismus 33 ein Stück aus der Öffnung 24 ausgeschoben werden und auch über eine Strecke von einigen Millimetern in Bezug auf den Rand der Öffnung 24 in das Innere des Werkzeuges zurückgezogen werden.

An der Oberfläche des Gehäuses ist mindestens ein elektrisch leitfähiger Körper vorgesehen, wobei ein solcher Körper z.B. durch das Gehäuserohr 37, durch einen Halteklipp 38 oder eine Abschlusskappe 39 gebildet sein kann und wobei man vorteilhaft an solchen elektrisch leitfähigen Körpern auch eine Möglichkeit zum Anschluss eines elektrischen Verbindungssteckers zum Anschluss einer Hilfselektrode vorsieht. Der elektrisch leitfähige Stift 25 ist dabei mit mindestens einem derartigen, an der Gehäuseoberfläche vorgesehenen, elektrisch leitfähigen Körper 37, 38, 39 über eine Schaltungsanordnung 27 verbunden, die zur Messung des elektrischen Widerstandswertes ausgebildet ist. Im Übrigen ist der Stift 25 im Werkzeug 21 elektrisch isoliert angeordnet, so dass keine direkte elektrisch leitende Verbindung zu den elektrisch leitfähigen Körpern 37, 38, 39 vorliegt und solcherart mit der Schaltungsanordnung 27 der Wert des elektrischen Widerstandes eines jeweils außen zwischen dem elektrisch leitfähigen Stift 25 und den elektrisch leitfähigen Körpern 37, 38, 39 anliegenden Körpers bzw. Stromweges erfasst werden kann. Die Schaltungsanordnung 27 ist mit einem optischen Indikator 41, der z.B. in Form einer Leuchtdiode ausgebildet ist, verbunden, wobei dieser Indikator für die Anzeige des Überschreitens eines bestimmten Widerstandswertes ausgebildet sein kann oder auch zur detaillierteren Angabe des jeweils vorliegenden Widerstandswertes durch Steuerung der Leuchtintensität oder durch Steuerung der Frequenz eines intermittierenden Aufleuchtens ausgebildet sein kann. In ähnlicher Weise kann man auch ergänzend zu einem optischen oder anstelle eines solchen einen akustischen Indikator vorsehen, wobei auch in diesem Fall durch Intensitätsvariation oder Tonhöhenvariation detailliertere Angaben über einen vorliegenden Widerstandswert oder einfach die Tatsache, dass ein bestimmter Grenzwert überschritten ist, angezeigt werden können.

Bei dem in den Fig. 13 und 14 dargestellten Handhabungswerkzeug 21 ist im Gehäuse 22 dieses Werkzeuges ein Tragkörper 55 vorgesehen, in dem eine Anzahl von Funktionsteilen dieses Werkzeuges angeordnet bzw. untergebracht sind. An dem in Fig. 12 links liegenden Ende dieses Tragkörpers befindet sich ein Einschnappsitz 58 für eine Anschlaghülse 59 und ein Einschnappsitz 60, zum Aufsetzen der das Vorderende des Gehäuses 22 bildenden Kappe 23, wobei an der Spitze dieser Kappe 23 die Öffnung 24 vorgesehen ist, durch die der elektrisch leitfähige Stift 25 über einen Teil seiner Länge ausschiebbar ist. Der Stift 25 ragt durch eine in der Anschlaghülse 59 vorgesehene Öffnung. Zur axialen Federung und zur Axialverschiebung des Stiftes 25 ist eine erste Feder 42, welche mit ihrem einen Ende 43 an der einen ersten Fixpunkt 44 bildenden Anschlaghülse 59 anliegt und mit ihrem anderen Ende 45 an einem Bund 46 des Stiftes 25 angreift, und weiter eine Reihenanordnung, die aus einer zweiten Feder 47 und dem Axialverschiebemechanismus 33 gebildet ist, vorgesehen, wobei ein Ende dieser Reihenanordnung an einem durch eine Wand 61 gebildeten zweiten Fixpunkt 49 im Tragkörper 55 sitzt und das andere Ende 50 dieser Reihenanordnung am Bund 46 des Stiftes 25 angreift. Der Axialverschiebemechanismus 33 weist dabei zwei Stützkörper 51, 52 auf, die in Axialrichtung 26 des Stiftes 25 auf diesen Stift folgend in axialer Aufeinanderfolge aneinander anliegend und gegeneinander verdrehbar im Tragkörper 55 angeordnet sind. Die Stützkörper 51, 52 weisen schräg zur Axialrichtung 26 verlaufende Flächen auf, wobei Schrägflächen des einen Stützkörpers 51 an Schrägflächen des anderen Stützkörpers 52 anliegen. Diese Schrägflächen sind in Form axial wirkender Verzahnungen ausgebildet, wobei durch verschiedene Höhen der Zähne eines Stützkörpers im Zuge einer relativen Verdrehung der beiden Stützkörper zueinander, diese beiden Stützkörper in alternierender Aufeinanderfolge relativ zueinander zwei verschiedene Axialpositionen einnehmen und solcherart die axiale Gesamtlänge dieser beiden Stützkörper 51, 52 durch zueinander relatives Verdrehen veränderbar ist. Es ist dabei einer dieser Stützkörper im Tragkörper 55 des Werkzeuges 21 drehfest angeordnet, während der andere Stützkörper verdrehbar angeordnet und mit dem außen am Gehäuse 22 angeordneten axial beweglichen Betätigungselement 34 zum Verdrehen dieses Stützkörpers verbunden ist. Der aus den Stützkörpern 51, 52 gebildete Axialverschiebemechanismus 33 ist im Tragkörper 55 geringfügig axial verschiebbar, und es ist damit der Stift 25 in seiner, ein Stück aus der Öffnung 24 ausgeschobenen Position gegen die Kraft der Feder 47 rückschiebbar, was zum selbsttätigen Abstreifen eines auf diesen Stift aufgeschobenen Ringplättchens beim Andrücken desselben an die Hautoberfläche genützt werden kann. Im dargestellten Fall ist in die Reihenanordnung, welche den Axialverschiebemechanismus 33 und die zweite Feder 47 enthält, auch eine Batterie 62 eingefügt, wobei die erwähnte zweite Feder 47 auch eine elektrische Verbindung zu einem Pol dieser Batterie 62 bildet. Der andere Pol der Batterie 62 liegt an einem Kontakt 63. Die Batterie 62 dient zur Speisung der auf der Schaltungsplatine 40 ausgebildeten Schaltungsanordnung 27, welche einerseits mit einer nicht näher dargestellten Leitung mit einem Anschlusskontakt 64 verbunden ist, der eine elektrische Verbindung zum Stift 25 herstellt, und andererseits mit dem elektrisch leitend ausgebildeten Gehäuserohr 37 in Verbindung steht. Weiters ist an die Schaltungsanordnung 27 die als optischer Indikator vorgesehene Leuchtdiode 41 angeschlossen, welche in einen transparenten Ring 65 eingesetzt ist, der bewirkt, dass das Leuchten der Diode 41 von allen Seiten her wahrgenommen werden kann.

Der Durchmesser des Stiftes 25 an seinem aus der Öffnung 24 ausschiebbaren Ende wird vorzugsweise gleich dem Innendurchmesser der mit dem Werkzeug 21 zu applizierenden Ringplättchen gewählt, damit diese Ringplättchen ohne zusätzliche Maßnahmen, nachdem sie aufgeschoben sind, auf diesem Stift verharren und auch wieder leicht vom Stift abgeschoben werden können. Weiter wird vorzugsweise zur Herstellung der Kappe ein geringfügig elastisch nachgiebiges Material gewählt, und der Durchmesser bzw. Querschnitt der Öffnung 24 entsprechend einem leichten Schiebesitz für das Einfügen von Erhebungen, die an der scheibenartigen Basis von Stimulationselektroden vorgesehen sind, gewählt.

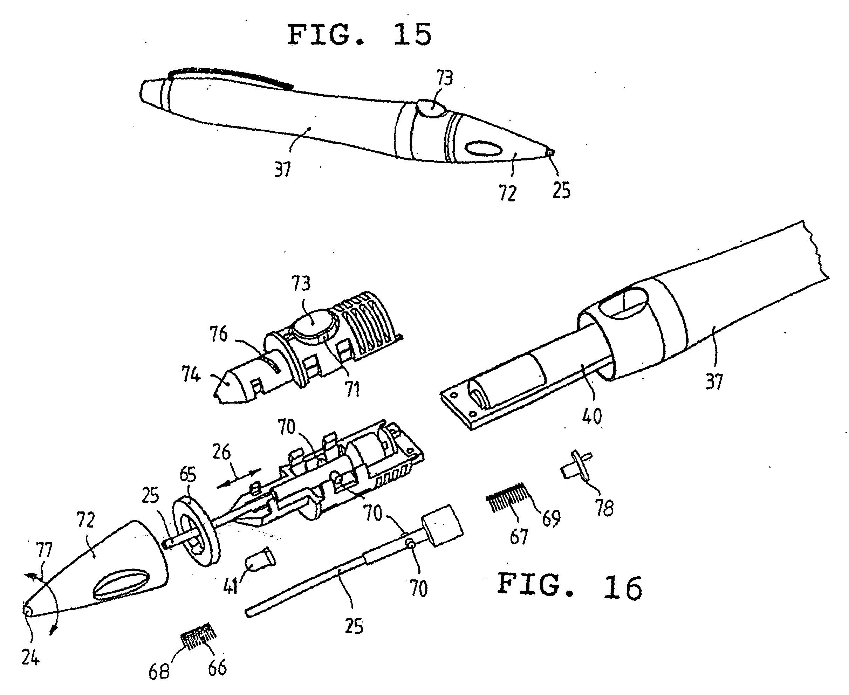

Die vorgenannte Wahl des Durchmessers des Stiftes 25 und des Durchmessers bzw. Querschnitts der Öffnung 24 wird vorteilhaft auch bei der in den Fig. 15 und 16 dargestellten Ausführungsform eines erfindungsgemäß ausgebildeten Handhabungswerkzeuges getroffen. Es ist bei dieser Ausführungsform der elektrisch leitfähige Stift 25 in einem zweiteiligen elektrisch isolierenden Lagerkörper 74, 75 axial verschiebbar angeordnet. Der Lagerkörper 74, 75 sitzt fix im elektrisch leitfähigen Gehäuserohr 37. Im Lagerkörper sind auch zwei in Axialrichtung 26 des Stiftes 25 wirkende Federn 66, 67 angeordnet, welche sich mit ihren Enden 68, 69 je an einem durch den Lagerkörper 74, 75 gebildeten Fixpunkt abstützen und mit ihren anderen Enden am Stift 25 angreifen. Am Stift 25 sind Ansätze 70 vorgesehen, die mit Schrägflächen zusammenwirken, welche an einem Schiebekörper 71 vorgesehen sind, der seinerseits mit einem Druckknopf 73, welcher aus dem Gehäuse ragt, integriert ist. Beim Betätigen des Druckknopfes 73 wird der Stift in Richtung eines Ausschiebens aus der Öffnung 24 bewegt. Die das Vorderende des Gehäuses bildende Kappe 72 ist axial versetzbar, wobei in der in Fig. 15 dargestellten Position der axial federnd gelagerte Stift 25 aus der Öffnung 24 ragt und so dieser Stift als Suchelektrode benützt und auf diesen Stift ein Ringplättchen zur Applikation aufgesteckt werden kann. Durch die federnde Lagerung des Stiftes 25 kann dieser in die Öffnung 24 gedrückt und damit das Ringplättchen an die Hautoberfläche appliziert werden. Durch axiales Versetzen der Kappe 72 in Richtung der Ausschiebebewegung des Stiftes 25 gelangt das vordere Ende des Stiftes in die Öffnung, und zwar bis zu einem Abstand von einigen Millimetern vom Öffnungsrand, und es kann dann eine Elektrode an der Öffnung 24 durch Einfügen einer an der Elektrode vorgesehenen Erhebung in die Öffnung fixiert werden. Durch Betätigung des Druckknopfes 73 kann der Stift 25 zur Öffnung 24 hin verschoben und damit die Elektrode zur Applikation aus der Öffnung 24 ausgeschoben werden. Die Kappe 72 weist bei der in Fig. 15 und 16 dargestellten Ausführungsform an ihrer Innenseite einen Zapfen auf, der in eine am Lagerkörper 74 vorgesehene Schrägnut 76 eingreift, und es führt die Kappe 72 damit beim Verdrehen gemäß Pfeil 77 eine diese Kappe 72 axial versetzende Schraubbewegung aus. Der Stift 25 ist bei dieser Ausführungsform über die Feder 67 und einen Kontakt 78 mit einer Platine verbunden, welche eine Schaltungsanordnung zur Messung des elektrischen Widerstandes und eine Batterie trägt. Diese Schaltungsanordnung steht auch mit einem Indikator in Form einer Leuchtdiode, die in einen Leuchtring eingesetzt ist, in Verbindung.

## Patentansprüche

1. Elektrodensystem (1) zur elektrischen Punktual-Stimulationstherapie mit mindestens einer bei unter der Hautoberfläche (2) befindlichen Rezeptorbereichen (3) anzuordnenden Punktual-Stimulationselektrode (4), welche eine scheibenartige Basis (5) aufweist, die an ihrer der Hautoberfläche (2) zuzuwendenden Seite (6), in der Mitte derselben, mit einer Nadel (7) oder Spitze versehen ist, über die eine Stromeinleitung in den zu stimulierenden Rezeptorbereich (3) erfolgt, wobei diese Punktual-Stimulationselektrode (4) über eine flexible Leitung (8) von einem Behandlungsstromgenerator gespeist wird, **dadurch gekennzeichnet, dass** zur Fixierung der jeweiligen Punktual-Stimulationselektrode (4) an der Hautoberfläche (2) ein vor dem Ansetzen bzw. Einstechen der Elektrode (4) an der Hautoberfläche (2) anzuordnendes Ringplättchen (9) vorgesehen ist, das mindestens an der der Hautoberfläche (2) zuzuwendenden Seite (10) eine zur Haftung an der Hautoberfläche vorgesehene Klebeschicht (11) trägt, und dass weiter eine Haftverbindung (13) zwischen der anderen Seite (12) dieses Ringplättchens (9) und der die Nadel (7) oder Spitze aufweisenden Seite (6) der Basis (5) der Punktual-Stimulationselektrode (4) vorgesehen ist.

2. Elektrodensystem nach Anspruch 1, **dadurch gekennzeichnet, dass** die scheibenartige Basis (5) der Punktual-Stimulationselektrode (4) an ihrer der Nadel (7) oder Spitze abgewandten Seite (14) eine zum passenden Einfügen in eine Aufnahmeöffnung eines Handhabungswerkzeuges und zum Aufsetzen einer elektrischen Verbindungsklemme vorgesehene Erhebung (15) aufweist.

3. Elektrodensystem nach Anspruch 2, **dadurch gekennzeichnet, dass** die an der Basis (5) der Punktual-Stimulationselektrode (4) vorgesehene Erhebung (15) einen ringsum laufenden ausgeprägten Seitenrand (16) aufweist, der durch zueinander parallele erzeugende Gerade, die annähernd senkrecht zur Basis (5) verlaufen, gebildet ist oder zumindest in Teilbereichen hinterschnitten geformt ist.

4. Elektrodensystem nach einem der Ansprüche 1 bis 3, **dadurch gekennzeichnet, dass** zur Bildung der Haftverbindung (13) zwischen dem Ringplättchen (9) und der Basis (5) der Punktual-Stimulationselektrode (4) auch an der der Punktual-Stimulationselektrode (4) zuzuwendenden Seite (12) des Ringplättchens (9) eine Klebeschicht (20) vorgesehen ist.

5. Elektrodensystem nach einem der Ansprüche 1 bis 3, **dadurch gekennzeichnet, dass** zur Bildung der Haftverbindung (13) zwischen dem Ringplättchen (9) und der Basis (5) der Punktual-Stimulationselektrode (4) an der dem Ringplättchen (9) zuzuwendenden Seite (6) der Basis (5) dieser Elektrode (4) eine Klebeschicht vorgesehen ist.

6. Elektrodensystem nach einem der vorhergehenden Ansprüche, **dadurch gekennzeichnet, dass** eine Überdeckung der jeweiligen Punktual-Stimulationselektrode (4), welche mit einem klebenden Ringplättchen (9), an der Hautoberfläche (2) fixiert ist, mit einer flexiblen, rings um diese Elektrode an der Hautoberfläche (2) haftenden Pflasterscheibe (18) vorgesehen ist.

7. Elektrodensystem nach Anspruch 6, **dadurch gekennzeichnet, dass** die Pflasterscheibe (18) einen annähernd radialen Einschnitt (19) zur Passage einer zum Behandlungsstromgenerator führenden Leitung (8) aufweist.

8. Handhabungswerkzeug zur Applikation eines Elektrodensystems nach einem der vorhergehenden Ansprüche an die Hautoberfläche, **gekennzeichnet durch** einen in einem Hohlstabgehäuse (22), das einem Handschreibgerät formähnlich und an seinem Vorderende zugespitzt geformt ist sowie an diesem Vorderende eine Öffnung (24) aufweist, axial verschiebbar gelagerten, elektrisch leitfähigen Stift (25), der über einen Teil seiner Länge ausschiebbar und **durch** die Öffnung (24) rückziehbar ist, und dessen Durchmesser an der am Vorderende des Gehäuses (22) vorgesehenen Öffnung (24) dem Innendurchmesser des Ringplättchens (9) entspricht, wobei zum Ausschieben und Rückziehen des elektrisch leitfähigen Stiftes (25) im Hohlstabgehäuse (22) ein mit mindestens einem am Hohlstabgehäuse vorgesehenen Betätigungselement (34) steuerbarer Axialverschiebemechanismus (33), mit dem dieser Stift (25) gekuppelt ist, angeordnet ist, wobei dieser Stift im Werkzeug (21) ohne direkte elektrisch leitende Verbindung zu an der Gehäuseoberfläche vorgesehenen elektrisch leitfähigen Körpern (37, 38, 39) angeordnet und über eine im Werkzeug (21) angeordnete Schaltungsanordnung (40) zur Messung des elektrischen Widerstandswertes mit einem solchen elektrisch leitfähigen Körper (37, 38, 39) verbunden ist, welche Schaltungsanordnung (40) den Wert des elektrischen Widerstandes des jeweils außen zwischen dem elektrisch leitfähigen Stift (25) und dem genannten elektrisch leitfähigen Körper (37, 38, 39) anliegenden Stromweges erfasst, und wobei diese Schaltungsanordnung (40) mit einem optischen und/oder akustischen Indikator verbunden ist.

9. Handhabungswerkzeug nach Anspruch 8 zur Applikation eines Elektrodensystems nach Anspruch 2 oder 3, **dadurch gekennzeichnet, dass** der Querschnitt der am Vorderende des Gehäuses des Handhabungswerkzeuges (21) vorgesehenen Öffnung (24) zum Einfügen der an der scheibenartigen Basis (5) der Punktual-Stimulationselektrode (4) vorgesehenen Erhabung (15) bemessen ist.

10. Handhabungswerkzeug nach Anspruch 8 oder 9, **dadurch gekennzeichnet, dass** der elektrisch leitfähige Stift (25) in Axialrichtung (26) federnd verschiebbar angeordnet ist.

11. Handhabungswerkzeug nach Anspruch 10, **dadurch gekennzeichnet, dass** zur axialen Federung und zur Axialverschiebung des axial verschiebbar gelagerten Stiftes (25) eine erste Feder (42), welche mit ihrem einen Ende (43) an einem ersten Fixpunkt (44) im werkzeug (21) sitzt und mit ihrem anderen Ende (45) an diesem Stift (25) angreift, und weiter eine aus einer zweiten Feder und einem Axialverschiebemechanismus (33) gebildete Reihenanordnung vorgesehen sind, wobei ein Ende dieser Reihenanordnung an einem zweiten Fixpunkt (49) im Werkzeug sitzt und das andere Ende (50) dieser Reihenanordnung am genannten Stift (25) angreift.

12. Handhabungswerkzeug nach einem der Ansprüche 8 bis 11, **dadurch gekennzeichnet, dass** der Axialverschiebemechanismus (33) zwei Stützkörper (51, 52) aufweist, die in Axialrichtung (26) des federnd axial verschiebbar gelagerten Stiftes (25) auf diesen Stift (25) folgend in axialer Aufeinanderfolge aneinander anliegend und gegeneinander verdrehbar angeordnet sind und schräg zur Axialrichtung (26) verlaufende flächen aufweisen, wobei Schrägflächen des einen Stützkörpers (51) an Schrägflächen des. anderen Stützkörpers (52) anliegen und wobei die axiale Gesamtlänge dieser beiden Stützkörper (51, 52) durch zueinander relatives Verdrehen veränderbar ist und einer dieser Stützkörper im Werkzeug (21) drehfest angeordnet ist, während der andere Stützkörper verdrehbar angeordnet und mit einem außen am Gehäuse (22) angeordneten Betätigungselement (34) zum Verdrehen dieses stützkörpers verbunden ist.

13. Handhabungswerkzeug nach Anspruch 11 oder Anspruch 12 mit Anspruch 11, **dadurch gekennzeichnet, dass** eine erste Feder (42), welche den axial verschiebbar gelagerten Stift (25) an seinem Vorderabschnitt (53) umgibt und mit ihrem einen Ende (43) an einem Fixpunkt (44) in der Nähe der Öffnung (24) sitzt und mit ihrem anderen Ende (45) am genannten Stift (25) angreift, vorgesehen ist, dass auf den hinteren Abschnitt (54) des Stiftes eine aus dem Axialverschiebemechanismus (33), einer Batterie (62) und einer zweiten Feder (47) gebildete Reihenanordnung folgt, wobei die zweite Feder (47) an einem zweiten Fixpunkt (49) im Werkzeug (21) sitzt und auch eine elektrische Verbindung zu einem Pol der Batterie (62) bildet.

14. Handhabungswerkzeug nach Anspruch 10, **dadurch gekennzeichnet, dass** am axial verschiebbar gelagerten Stift (25) zwei axial wirkende Federn (66, 67) angreifen, welche sich mit ihren der jeweiligen Angriffsstelle am Stift (25) abgewandten Enden (68, 69) je an einem gegen Axialverschiebung festliegenden Fixpunkt im Gehäuse abstützen, dass der Axialverschiebemechanismus durch einen am Stift (25) seitlich abstehenden Ansatz (70) und einen quer zur Axialrichtung (26) des Stiftes (25) bewegbaren schiebekörper (71) gebildet ist, wobei am Ansatz (70) und/oder am Schiebekörper (71) mindestens eine Schrägfläche vorgesehen ist, durch welche der Stift (25) bei einer quer zur Axialrichtung (26) des Stiftes (25) erfolgenden Bewegung des Schiebekorpers (71) im Zusammenwirken von Ansatz (70) und Schiebekörper (71) aus einer Ruhelage in Richtung eines Ausschiebens aus der Öffnung (24) bewegbar ist, und dass das vorderende des Gehäuses (22) des Werkzeuges (21) durch eine gegenüber dem übrigen Gehäuseabschnitt (37) axial versetzbar angeordnete Kappe (72) gebildet ist, welche durch diese axiale Versetzung die axiale Relativlage der Öffnung (24) und des Vorderendes des Stiftes (25) dahingehend veränderbar ist, dass in einer Position der Kappe (72) der in Ruhelage befindliche Stift (25) ein Stück aus der Öffnung (24) ragt und in einer anderen Position der Kappe (72) der in Ruhelage befindliche Stift (25) ein Stück gegenüber dem Außenrand der Öffnung (24) zurückgezogen ist.

15. Handhabungswerkzeug nach Anspruch 14, dadaurrh gekennzeichnet, dass die axial versetzbar angeordnete Kappe (72) zur Axialversetzung gegenüber dem übrigen Gehäuseabschnitt (37) schraubbar angeordnet ist.

16. Handhabungswerkzeug nach Anspruch 14 oder 15, **dadurch gekennzeichnet, dass** der Schiebekörper mit einem das Betätigungselement bildenden Druckknopf (73) versehen ist.

17. Handhabungswerkzeug nach einem der Ansprüche 8 bis 11, **dadurch gekennzeichnet, dass** als Axialverschiebemechanismus ein elektromotorisch oder elektromagnetisch angetriebenes Verschiebegetriebe vorgesehen ist, welches über elektrische Leichtgangtasten oder Sensorfelder steuerbar ist.

## Claims

1. An electrode system (1) for an electric punctual stimulation therapy, comprising at least one punctual stimulation electrode (4) to be arranged at receptor regions (3) located below the skin surface (2), which punctual stimulation electrode comprises a disk-like base (5) which, in the middle thereof, at its side (6) that is to face the skin surface (2), is provided with a needle (7) or tip via which an introduction of current into the receptor region (3) to be stimulated occurs, this punctual stimulation electrode (4) being fed from a treatment current generator via a flexible line (8), **characterised in that** for fixing the respective punctual stimulation electrode (4) at the skin surface (2), an annular platelet (9) is provided which is to be arranged on the skin surface (2) prior to a setting of the electrode (4) thereto or piercing thereof, which annular platelet has an adhesive layer (11) at least at its side (10) to face the skin surface (2) for an adhesion to the skin surface and that, furthermore, an adhesive connection (13) is provided between the other side (12) of this annular platelet (9) and the side (6) of the base (5) of the punctual stimulation electrode (4) that comprises the needle (7) or tip.

2. An electrode system according to claim 1, **characterised in that** the disk-like base (5) of the punctual stimulation electrode (4) at its side (14) facing away from the needle (7) or tip has an elevation (15) provided for fitting insertion in a receiving aperture of a handling tool and for putting on of an electric connecting terminal.

3. An electrode system according to claim 2, **characterised in that** the elevation (15) provided at the base (5) of the punctual stimulation electrode (4) has a peripherally extending pronounced lateral rim (16), which rim is formed by generatrix-straight lines extending in parallel to each other and approximately perpendicularly to the base (5), or which rim is formed to be undercut at least in partial regions thereof.

4. An electrode system according to any one of claims 1 to 3, **characterised in that** for forming the adhesive connection (13) between the annular platelet (9) and the base (5) of the punctual stimulation electrode (4), an adhesive layer (20) is also provided on that side (12) of the annular platelet (9) which is to face the punctual stimulation electrode (4).

5. An electrode system according to any one of claims 1 to 3, **characterised in that** for forming the adhesive connection (13) between the annular platelet (9) and the base (5) of the punctual stimulation electrode (4), an adhesive layer is provided on that side (6) of the base (5) of this electrode (4) which is to face the annular platelet (9).

6. An electrode system according to any one of the preceding claims, **characterised in that** a cover of the respective punctual stimulation electrode (4) that is fixed to the skin surface (2) by means of an adhesive annular platelet (9), is provided with a flexible adhesive tape disk (18) adhering to the skin surface (2) all around this electrode.

7. An electrode system according to claim 6, **characterised in that** the adhesive tape disk (18) has an approximately radial incision (19) for the passage of a line (8) leading to the treatment current generator.

8. A handling tool for applying an electrode system according to any one of the preceding claims to the skin surface, **characterised by** an electrically conductive pin (25) that is axially displaceably mounted in a hollow rod housing (22) of a shape similar to a hand writing tool and tapers to a tip at its front end and has an aperture (24) at this front end, which pin can be pushed out by part of its length and is retractable through this aperture (24), the pin having a diameter at the aperture (24) provided at the front end of the housing (22) which corresponds to the inner diameter of the annular platelet (9), wherein an axial displacement mechanism (33) is arranged in the hollow rod housing (22) and is controllable by at least one actuating element (34) provided on the hollow rod housing (22), and is coupled to this pin (25) for pushing out and retracting the latter, wherein this pin is arranged in the tool (21) without a direct electrically conductive connection to electrically conductive members (37, 38, 39) provided on the housing surface, and is connected to one such electrically conductive member (37, 38, 39) via a circuit arrangement (40) arranged in the tool (21) for measuring the electric resistance value, which circuit arrangement (40) senses the value of the electric resistance of the respective current path which externally is applied between the electrically conductive pin (25) and the said electrically conductive member (37, 38, 39), and wherein this circuit arrangement (40) is connected to an optic and/or acoustic indicator.

9. A handling tool according to claim 8 for applying an electrode system according to claim 2 or 3, **characterised in that** the cross-section of the aperture (24) provided at the front end of the housing of the handling tool (21) is dimensioned for insertion of the elevation (15) provided on the disk-like base (5) of the punctual stimulation electrode (4).

10. A handling tool according to claim 8 or 9, **characterised in that** the electrically conductive pin (25) is arranged to be resiliently displaceable in axial direction (26).

11. A handling tool according to claim 10, **characterised in that** for an axial resilience and for the axial displacement of the axially displaceably mounted pin (25), a first spring (42) which is seated with its one end (43) at a first fixed point (44) in the tool (21) and with its other end (45) engages this pin (25), and furthermore, a serial arrangement formed of a second spring and an axial displacement mechanism (33) is provided, one end of this serial arrangement being seated at a second fixed point (49) in the tool, and the other end (50) of this serial arrangement engaging said pin (25).

12. A handling tool according to any one of claims 8 to 11, **characterised in that** the axial displacement mechanism (33) has two supporting bodies (51, 52) arranged in axial direction (26) of the resiliently axially displaceably mounted pin (25) following upon this pin (25) and abutting each other in axial sequence and rotatable relative to each other and comprising surfaces that extend obliquely to the axial direction (26), oblique surfaces of the one supporting body (51) contacting oblique surfaces of the other supporting body (52), and the axial total length of these two supporting bodies (51, 52) being changeable by relative rotation thereof, and one of these supporting bodies being non-rotationally arranged in the tool (21), while the other supporting body is rotatably arranged and connected to an actuating element (34) arranged externally on the housing (22) for rotation of this supporting body.

13. A handling tool according to claim 11 or to claim 12 in combination with claim 11, **characterised in that** a first spring (42) is provided which surrounds the axially displaceably mounted pin (25) at its front portion (53) and with its one end (43) is seated on a fixed point (44) in the vicinity of the aperture (24) and with its other end (45) engages the said pin (25), that on the rear portion (54) of the pin a serial arrangement follows, formed of the axial displacement mechanism (33), a battery (62) and a second spring (47), the second spring (47) being seated on a second fixed point (49) in the tool (21) and also forming an electric connection to one pole of the battery (62).

14. A handling tool according to claim 10, **characterised in that** two axially acting springs (66, 67) engage the axially displaceably mounted pin (25), which springs are each supported with their ends (68, 69) facing away from the respective site of engagement on pin (25), on a fixed point in the housing that is fixed against an axial displacement, that the axial displacement mechanism is formed by a nose (70) laterally projecting from the pin (25) and a shifting body (71) transversely movable to the axial direction (26) of the pin (25), with at least one oblique surface being provided on the nose (70) and/or on the shifting body (71), by which the pin (25), at a movement of the shifting body (71) transversely to the axial direction (26) of the pin (25), in cooperation of nose (70) and shifting body (71), is movable from a resting position in the direction of a pushing out of the aperture (24), and that the front end of the housing (22) of the tool (21) is formed by a cap (72) arranged such that it can be axially offset relative to the remaining housing portion (37), wherein by this axial offsetting, the axial relative position of the aperture (24) and the front end of the pin (25) is changeable such that in one position of the cap (72), the pin (25) which is in its resting position, projects a little from the aperture (24), and in another position of the cap (72) the pin (25) which is in its resting position is retracted a little relative to the outer rim of the aperture (24).

15. A handling tool according to claim 14, **characterised in that** the axially offsettably arranged cap (72) is screwably arranged for said axial offsetting relative to the remaining housing portion (37).

16. A handling tool according to claim 14 or 15, **characterised in that** the shifting body is provided with a push-button (73) forming the actuating element.

17. A handling tool according to any one of claims 8 to 11, **characterised in that** a displacement gear driven by an electromotor or a solenoid means is provided as the axial displacement mechanism, which displacement gear can be controlled via easy-movable electric buttons or sensor fields.

## Revendications

1. Système (1) d'électrodes destiné à la thérapie par stimulation électrique ponctuelle comportant au moins une électrode de stimulation ponctuelle (4) qui doit être disposée dans des zones de récepteur (3) situées au-dessous de la surface (2) de la peau, et qui porte une base en forme de disque (5), qui est équipée, au niveau de sa face (6) tournée vers la surface (2) de la peau, au milieu de cette face, d'une aiguille (7) ou d'une pointe, à l'aide de laquelle s'effectue une entrée de courant dans la zone de récepteur à stimuler (3), cette électrode de stimulation ponctuelle (4) étant alimentée par un générateur de courant de traitement par l'intermédiaire d'une ligne flexible (8), **caractérisé en ce que** pour la fixation de l'électrode de stimulation ponctuelle respective (4) sur la surface (2) de la peau, il est prévu une plaquette annulaire (9), qui doit être disposée pendant l'application ou l'enfichage de l'électrode (4) dans la surface (2) de la peau et qui porte, au moins sur la face (10) tournée vers la surface (2) de la peau, une couche d'adhésif, qui est destinée à adhérer à la surface de la peau et qu'en outre il est prévu une liaison adhésive (13) entre l'autre face (12) de cette plaquette annulaire (9) et la face (6), portant l'aiguille (7) ou la pointe, de la base (5) de l'électrode de stimulation ponctuelle (4).

2. Système d'électrodes selon la revendication 1, **caractérisé en ce que** la base en forme de disque (5) de l'électrode de stimulation ponctuelle (4) comporte, sur sa face (14) tournée à l'opposé de l'aiguille (7) ou de la pointe, une partie surélevée (15) prévue pour une insertion ajustée dans une ouverture de réception d'un outil de manipulation et pour le montage d'une bande électrique de liaison.

3. Système d'électrodes selon la revendication 2, **caractérisé en ce que** la partie surélevée (15), qui est prévue sur la base (5) de l'électrode de stimulation ponctuelle (4), comporte un rebord latéral saillant circonférentiel (16), qui est formé par deux droites parallèles entre elles, qui s'étendent approximativement perpendiculairement sur la base (5), ou est formée en dépouille au moins dans des zones partielles.

4. Système d'électrodes selon l'une des revendications 1 à 3, **caractérisé en ce que** pour la formation de la liaison d'adhérence (13) entre la plaquette annulaire (9) et la base (5) de l'électrode de stimulation ponctuelle (4) il est également prévu une couche adhésive (20) sur la face (12), tournée vers une électrode de stimulation ponctuelle (4), de la plaquette annulaire (9).

5. Système d'électrodes selon l'une des revendications 1 à 3, **caractérisé en ce que** pour la formation de la liaison adhésive (13) entre la plaquette annulaire (9) et la base (5) de l'électrode de stimulation ponctuelle (4), une couche adhésive est prévue sur la face (6), tournée vers la plaquette annulaire (9), de la base (5) de cette électrode (15).

6. Système d'électrodes selon l'une des revendications précédentes, **caractérisé en ce qu'**il est prévu un revêtement de l'électrode de stimulation ponctuelle respective (4) qui est fixé par une plaquette annulaire adhésive (9) à la surface (2) de la peau, comportant un disque de dallage souple (18) qui adhère annulairement autour de cette électrode à la surface (2) de la peau.

7. Système d'électrodes selon la revendication 6, **caractérisé en ce que** le disque de dallage (18) comporte une encoche approximativement radiale (19) pour le passage d'une ligne (8) conduisant au générateur de courant de traitement.

8. Outil de manipulation pour l'application d'un système d'électrodes selon l'une des revendications précédentes, sur la surface de la peau, **caractérisé par** une tige électriquement conductrice (25), qui est montée de manière à être déplaçable axialement dans un boîtier de barreau creux (22), qui est agencé de manière à ressembler à un ustensile d'écriture manuel et est pointu au niveau de son extrémité avant et comporte une ouverture (24) sur son extrémité avant, la tige pouvant être ressortie sur une partie de sa longueur et rétractée à travers l'ouverture (24), et son diamètre au niveau de l'ouverture (24) prévue sur l'extrémité avant du boîtier (22) correspondant au diamètre intérieur de la plaquette annulaire (9), auquel cas pour déployer et rétracter la tige électriquement conductrice (25) dans le boîtier en forme de barreau creux (22) est disposé un mécanisme de déplacement axial (33), qui peut être commandé à l'aide d'au moins un élément d'actionnement (34) prévu sur le boîtier en forme de barreau creux, et auquel cette tige (25) est accouplée, cette tige étant disposée dans l'outil (21) sans liaison électriquement conductrice directe avec des corps électriquement conducteurs (37, 38, 39) prévus sur la surface de boîtier, et étant reliée, par l'intermédiaire d'un montage (40) disposé dans l'outil (21) et servant à mesurer la valeur résistive électrique, à un tel corps électriquement conducteur (37, 38, 39), lequel montage (40) détecte la valeur de la résistance électrique de la tige et de la voie de courant qui est située respectivement extérieurement entre la tige électriquement conductrice (25) et le corps électriquement conducteur indiqué (37, 38, 39), ce montage (40) étant relié à un inducteur à courant optique et/ou acoustique.

9. Outil de manipulation selon la revendication 8, pour l'application d'un système d'électrodes selon la revendication 2 ou 3, **caractérisé en ce que** la section transversale de l'ouverture (24) prévue sur l'extrémité avant du boîtier de l'outil de manipulation (21) est dimensionnée pour permettre l'insertion de la partie surélevée (15) prévue sur la base en forme de disque (5) de l'électrode de stimulation ponctuelle (4).

10. Outil de manipulation selon la revendication 8 ou 9, **caractérisé en ce que** la tige électriquement conductrice (25) est disposée de manière à être déplaçable électriquement dans la direction axiale (26).

11. Outil de manipulation selon la revendication 10, **caractérisé en ce que** pour la suspension axiale élastique et pour le déplacement axial de la tige (5) montée de manière à être déplaçable axialement, il est prévu un premier ressort (42), qui prend appui, par l'une de ses extrémités (43) au niveau d'un premier point de fixation (44) dans l'outil (21) et attaque, par son autre extrémité (45), cette tige (25), et en outre un dispositif en série formé par un second ressort et le mécanisme de déplacement axial (33), une extrémité de ce dispositif en série étant en appui au niveau d'un second point de fixation (49) dans l'outil, tandis que l'autre extrémité (50) de ce dispositif en série attaque la tige indiquée (25).

12. Outil de manipulation selon l'une des revendications 8 à 11, **caractérisé en ce que** le mécanisme de déplacement axial (33) comporte deux corps d'appui (51, 52), qui sont disposés dans la direction axiale de la tige (25) montée de manière à être déplaçable élastiquement, sur cette tige (25) à la suite de cette dernière selon une succession axiale et sont disposés de manière à pouvoir pivoter l'un par rapport à l'autre et possèdent des surfaces qui s'étendent obliquement par rapport à la direction axiale (26), des surfaces obliques d'un corps d'appui (52) s'appliquant sur des surfaces obliques de l'autre corps d'appui (51), et la longueur axiale totale de ces deux corps d'appui (51, 52) étant modifiable au moyen d'une torsion relative, et l'un de ces corps d'appui étant monté solidairement en rotation dans l'outil (21), tandis que l'autre corps d'appui est monté de manière à pouvoir tourner et est relié à un élément d'actionnement (34) disposé extérieurement sur le boîtier (22) pour faire tourner ce corps d'appui.

13. Outil de manipulation selon la revendication 11 ou la revendication 12 associée à la revendication 11, **caractérisé en ce qu'**un premier ressort (42), qui entoure la tige montée de manière à être déplaçable axialement (25) au niveau de sa section avant (53) et, par son autre extrémité (45) attaque la tige indiquée (25), qu'à la section arrière (54) de la tige succède un dispositif en série formé du mécanisme de déplacement axial (33) d'une batterie (42) et d'un second ressort (47), le second ressort (47) prenant appui au niveau d'un second point de fixation (49) dans l'outil (21) et établissant également une liaison électrique avec un pôle de la batterie (62).

14. Outil de manipulation selon la revendication 10, **caractérisé en ce que** sur la tige (25) montée de manière à être déplaçable axialement est attaquée par deux ressorts (66, 67), qui agissent axialement et qui prennent appui, par leurs extrémités (68, 69), tournées à l'opposé du point d'attaque respectif sur la tige (25), respectivement en des points fixes respectifs fixés contre tout déplacement axial, dans le boîtier, que le mécanisme de déplacement axial est formé par un appendice saillant (70), qui fait saillie latéralement sur la tige (25), et par un corps coulissant déplaçable transversalement par rapport à la direction axiale (26) de la tige (21), auquel cas sur l'appendice saillant (70) et/ou sur le corps coulissant (71) est prévue au moins une surface oblique, au moyen de laquelle la tige (25) peut être déplacée depuis une position de repos dans le sens d'un dégagement hors de l'ouverture (24) dans le cas d'un déplacement du corps coulissant (71), qui s'effectue transversalement par rapport à la direction (26) de la tige (25), lors de la coopération de l'embout saillant (70) et du corps coulissant (71) et que l'extrémité avant du boîtier (22) de l'outil (21) est formée par un capuchon (72) qui est disposé de manière à être déplaçable axialement par rapport au reste de la section de boîtier (37) et qui peut modifier, sous l'effet de ce déplacement axial, la position axiale relative de l'ouverture (24) et de l'extrémité avant de la tige (25) en ce sens que dans une position du capuchon (72), la tige (25) située dans la position de repos fait saillie légèrement hors de l'ouverture (24) et, lorsque le capot (72) est dans une autre position, la tige (25) située dans la position de repos est rétractée légèrement par rapport à la paroi de l'ouverture extérieure (24).

15. Outil de manipulation selon la revendication 14, **caractérisé en ce que** le capuchon (72) disposé de manière à pouvoir être décalé axialement est monté de manière à pouvoir être vissé dans son décalage axial par rapport à la partie restante (37) du boîtier.

16. Outil de manipulation selon la revendication 14 ou 15, **caractérisé en ce que** le corps coulissant est pourvu d'un bouton-poussoir (73) formant l'élément d'actionnement.

17. Outil de manipulation selon l'une des revendications 8 à 11, **caractérisé en ce qu'**il est prévu comme mécanisme de déplacement axial, un mécanisme de déplacement entraîné par un moteur électrique ou voie électromagnétique et qui peut être commandé par l'intermédiaire des touches électriques souples ou des champs de capteurs.
